# EUROPEAN PATENT SPECIFICATION

(11) **EP 2 125 530 B1**
(45) Date of publication and mention of the grant of the patent: **11.12.2013**
(21) Application number: 08724118.8
(22) Date of filing: 10.03.2008
(51) Int. Cl.: B65B 55/10, A61L 2/10, A61L 2/18, A61L 2/20, B65B 55/08

(54) **A METHOD AND DEVICE FOR PRODUCING A PACKAGING CONTAINER**
VERFAHREN UND VORRICHTUNG ZUR HERSTELLUNG EINES VERPACKUNGSGEBINDES
PROCÉDÉ ET DISPOSITIF POUR PRODUIRE UN RÉCIPIENT DE CONDITIONNEMENT

(30) Priority: 13.03.2007 SE 0700618
(43) Date of publication of application: 02.12.2009
(73) Proprietor: Tetra Laval Holdings & Finance S.A., 1009 Pully (CH)
(72) Inventor: ANDERSSON, Mats, S-262 52 Ängelholm (SE); SCHELLENBERG, Helen, S-240 36 Stehag (SE); MÅNSSON, Carl-Erik, S-241 91 Eslöv (SE); NELANDER, Kristian, S-244 33 Kävlinge (SE); JARTELIUS, Monica, S-212 42 Malmö (SE)
(74) Representative: Sundell, Hakan Oskar Anders
(86) International application number: PCT/SE2008/000186
(87) International publication number: WO 2008/111893

(56) References cited:
- EP-A2- 1 046 585
- WO-A1-99/21593
- US-A1- 2006 229 225
- MARQUIS R.E. ET AL.: 'Sporicidal interactions of ultraviolet irradiation and hydrogen peroxide related to aseptic technology' CHEMICAL ENGINEERING AND PROCESSING vol. 46, no. 6, June 2007, pages 547 - 553, XP005911056

## Description

### TECHNICAL FIELD

The present invention relates to a method of producing a packaging container with very low microbiological load by treating the packaging material in a filling machine with a combination of a chemical sterilization agent and UV-light. The invention also relates to a device in a filling machine for producing a packaging container with very low microbiological load, said device being adapted to treat the packaging material with a combination of a chemical sterilization agent and UV-light. Further, the invention relates to a filling machine provided with said device.

### BACKGROUND ART

Packaging containers, for packing different types of consumer products, e.g. foods such as milk or juice, may be of the type which has been manufactured from a packaging laminate comprising a core layer of paper or paperboard and one or more barrier layers of, for example, thermoplastic and aluminium foil.

One common packaging type is manufactured in a packing and filling machine in that a web of packaging laminate is divided into sheets, each sheet being reformed and sealed into a tubular blank which is closed in its one end in that an end wall in the form of a top of thermoplastic is injection moulded directly on the end portion. Thereafter, the packaging container is filled with the desired contents and closed and sealed at its other end. The result will be a packaging container which the applicant markets under the trademark Tetra Top®.

Such manufacture may also take as its starting point flat-laid, tubular blanks which, in the filling machine, are raised and initially sealed in their one end by folding and sealing, for forming a planar bottom surface. After filling with the desired contents, the packaging container is thereafter closed and sealed in a similar operation in its other end, but then for the formation of a top of gable-top type. The applicant markets such packaging containers under the trademark Tetra Rex®.

Today, packaging containers of the above-mentioned or similar type are normally used for packing, for example, milk and acidic products (pH below 4.2), such as, for example, juice, fruit drinks and sport drinks. The filled and closed packaging container is kept in cold storage, for example at a maximum of +8°C and, in such instance, has a shelf-life of between six and eight days. This shelf-life is generally sufficient in the packing of perishable goods and in handling in an existing, unbroken refrigerated chain, i.e. immediate refrigeration after filling and sealing, transport in refrigerated vehicles, as well as exposure in refrigerated product counters.

However, in order to extend the shelf-life of these products, it is possible to introduce a microorganism-reducing treatment in the filling machine. One example of this is described in the U.S. Patent No. 6,338,235. The treatment comprises exposing, in a first station, the packaging container blank to a microorganism-reducing treatment in that a sterilizing chemical substance in gas- or vapour form is supplied to the inside of the packaging container blank. Preferably, hydrogen peroxide is used at a concentration of between 0.5 and 1 %, which, after heating to a temperature of approx. 200°C, is supplied to the packaging container blank by means of a spray nozzle which finely divides the hydrogen peroxide. The hydrogen peroxide condenses on the inside of the packaging container blank which, as a result, is coated with a thin layer of sterilization agent. The packaging container blank is thereafter moved to a subsequent station where it is subjected to irradiation, which is preferably carried out by UV-light at a suitable, microorganism exterminating wavelength from a conventional UV-lamp. The UV-lamp can be wholly or partly lowered down into the packaging container, or alternatively be placed above the packaging container and, in such instance, use a reflector to ensure that the light beams reach all interior parts of the packaging container.

Combining a chemical sterilization agent with UV-light gives, in a per se known manner, a synergy effect which makes for an efficient extermination of microorganisms. This is used today in the vast majority of filling machines which manufacture the packaging container which the Applicant markets under the trademark Tetra Top®.

The advantages inherent in this type of treatment is that it is rapid and that it makes it possible to keep to a low concentration of the sterilization agent employed, which is positive from both the economical and environmental viewpoints.

One limitation, and one which has set out the limit for shelf-life, has however been the very occasional occurrence and multiplication of mould spores on the packaging laminate. These spores may contaminate the packaging material on, for example, storage in certain environments, and it has proved to be more difficult to exterminate them than most other microorganisms.

More recent research which int. al. is described in an article entitled Sporicidal interactions of ultraviolet irradiation and hydrogen peroxide related to aseptic technology written by J. D. Baldeck and R. E. Marquis, published in Chemical Engineering and Processing, 2006, demonstrates that the application of the chemical sterilization agent and the subsequent UV-light treatment can be separated, and that it is possible to create the above mentioned synergy effect also as long as up to 24 hours after the application of the chemical sterilization agent.

Practical trials in filling machines have recently demonstrated that this may be put to use so as to realise a considerably improved shelf-life for products packed in accordance with that described in the U.S. Patent No. 6,338,235. In such instance, it has proved that microorganisms in the form of mould spores can also be dealt with in a reliable manner. For certain products, for instance high acid products, it has even proved that the microbiological load will be so low that distribution can be put into effect in a non-refrigerated distribution chain.

### SUMMARY OF THE INVENTION

As is apparent from the background art discussion, the object of the present invention is to realise a method of producing a packaging container with very low microbiological load, by rendering considerably more efficient an existing microorganism-reducing treatment in a filling machine so that int. al. microorganisms in the form of mould spores can be exterminated reliably, at the same time as the concentration of chemical sterilization agent is kept at a continued low level. The above-mentioned object is attained by a method comprising the step of treating the packaging material in a filling machine with a combination of a chemical sterilization agent and UV-light, where a first partial quantity of the chemical sterilization agent is applied on the packaging material a sufficiently long time before the packaging material is irradiated with UV-light in order that a predetermined portion of expected microorganisms will have time to absorb a sufficient amount of the chemical sterilization agent in order to be exterminated on being irradiated with UV-light, and where a second partial quantity of the chemical sterilization agent is applied to the packaging material in a sterile chamber before the packaging material is irradiated with UV-light. By increasing the time elapsed from the application of a part of the chemical sterilization agent until such time as the packaging material is irradiated with UV-light, it is possible to ensure that any microorganisms which may possibly be present, above all mould spores, will have time to absorb a sufficient quantity of the sterilization agent to be able to be exterminated when the material is subsequently irradiated with UV-light. Many mould spores are hydrophobic and, as a result, it takes a longer time to wet them as compared with other micro-organisms, i.e. it takes a longer time to make sure that a sufficient number of hydrogen peroxide molecules are present near the spore membrane. It would thereby in principle apply to a filling machine that, the longer the time which elapses between the application of the sterilization agent in low concentration and the irradiation with UV-light, the more effective will be the extermination of the microorganisms.

In the filling machine described in the U.S. Patent No. 6,338,235, an early application of a part of the chemical sterilization agent may increase the time during which the microorganisms are exposed to the sterilization agent from the present time of approx. 1 second to approx. 40 seconds. Naturally, this affords a considerably greater possibility for the microorganisms to absorb the sterilization agent, which results in a more efficient extermination, and which in turn leads to an increased shelf-life for the product in the finished packaging container.

One currently preferred embodiment of the method according to the present invention is characterised in that the application of said first partial quantity of the chemical sterilization agent takes place outside the sterile chamber. Today, the entire dose of sterilization agent is applied in a first station in the sterile chamber in the filling machine. In a subsequent station in the sterile chamber, the material is subsequently irradiated. One method of increasing the exposure time between the chemical sterilization agent and microorganisms which occur could be to physically increase the distance between the stations involved. However, this would result in a physical extension of the sterile chamber, a feature which is only economically viable and practically applicable up to a certain limit. Instead, the application of the sterilization agent can be divided into two stations, of which one of these stations is positioned outside the sterile chamber. The first station may then be seen as a pre-treatment station where treatment of already existing microorganisms such as mould spores is commenced, while the second station, inside the sterile chamber, ensures that those microorganisms which may possibly land on the material on processing thereof in the machine are also dealt with.

Yet a further currently preferred embodiment of the method according to the invention is characterised in that the application of said first partial quantity of the chemical sterilization agent takes place in the very first, or one of the first, stations in which the packaging material is treated when it is conveyed through the filling machine. By such means, the previously described exposure time is maximised most simply.

In another currently preferred embodiment of the method according to the invention, this is realised purely practically in that the filling machine is loaded with at least one magazine reel of web-shaped packaging material, and that said first partial quantity of the sterilization agent is applied to the packaging material in the very first, or one of the very first, stations immediately after unwinding of the packaging material from the magazine reel. In initially reel-fed systems, this is a simple and efficient way of reducing the method according to the present invention into practice.

Further preferred embodiments are apparent from appended subclaims 4 to 12.

The object is also solved by a device according to the present invention, said device being adapted to treat the packaging material in a filling machine with a combination of a chemical sterilization agent and UV-light. The device is characterised in that it comprises means for applying a first partial quantity of the chemical sterilization agent to the packaging material, and a sterile chamber comprising means for applying a second partial quantity of the chemical sterilization agent to the packaging material and means for irradiating the packaging material with UV-light, whereby the means for applying the first partial quantity of the chemical sterilization is positioned away from the UV-light irradiating means so that a sufficiently long time will have past before the packaging material is irradiated with UV-light in order for a predetermined portion of expected micro-organisms to have absorbed a sufficient amount of the chemical sterilization agent so as to be exterminated when irradiated with the UV-light.

In a presently preferred embodiment of the device said means for applying the first partial quantity of the chemical sterilization agent is positioned outside the sterile chamber.

In a further presently preferred embodiment said means for applying the first partial quantity of the chemical sterilization agent is positioned in the very first or one of the first stations in which the packaging material is treated when conveyed through the filling machine.

In a yet further presently preferred embodiment the filling machine is supplied with at least one magazine reel of web-shaped packaging material and said means for applying the first partial quantity of the sterilization agent to the packaging material is positioned in the very first or one of the first stations immediately after the unwinding of the packaging material from the magazine reel.

In an additional presently preferred embodiment said means for applying a first partial quantity of sterilization agent is a bath of sterilization agent through which the packaging material runs, which bath is provided with means adapted to remove surplus sterilization agent when the packaging material emerges from the bath.

In yet another presently preferred embodiment the filling machine is supplied with flat-laid, tubular packaging blanks and said means for applying the first partial quantity of the sterilization agent is positioned in a station located at least downstream of a station for raising said packaging blank, and that said means is adapted to apply said sterilization agent to the inside of each said packaging blank.

In a further presently preferred embodiment said means for applying the first partial quantity of sterilization agent comprises means for vaporising the sterilization agent and means for spraying the sterilization agent in gaseous form onto at least that side of the packaging material which is to form the inside of the future packaging container.

In a further presently preferred embodiment said means for applying the second partial quantity of sterilization agent on the packaging material is positioned in the sterile chamber in a station substantially immediately prior to that station in which the packaging material is irradiated with UV-light.

In a presently preferred embodiment the sterilization agent is hydrogen peroxide (H₂O₂) in a concentration in the range of between 0.1 and 5 weight percent.

In yet a presently preferred embodiment the UV-light which is employed has a wavelength selected within the range of between 200 and 280 nanometres.

In a presently preferred embodiment said means for applying the first partial quantity of sterilization agent is enclosed in a chamber which is connected to a sterilization agent condensation and waste system comprising means for condensing the sterilization agent and means for diluting it.

The invention also involves a filling machine comprising a device as described above. Said filling machine is supplied with at least one magazine reel of web-shaped packaging material. Said means for applying the first partial quantity of sterilization agent to the inside of the packaging material is positioned in a station located downstream of a station for unwinding the packaging material from the magazine reel. The machine is provided with means for cutting the web-shaped packaging material into sheets and means for forming each sheet into a tubular packaging container blank. It is further provided with means for closing and sealing the one open end of the tubular packaging container blank by injection moulding a thin-walled top of thermoplastic on said end. The machine is also provided with means for conveying the packaging container blank into the sterile chamber. Said means for applying the second partial quantity of sterilization agent to the inside of the packaging container blank and the means for irradiating with UV-light are positioned in the sterile chamber, and said sterile chamber being further provided with means for filling the packaging container blank with the desired contents and means for sealing the open second end of the packaging container blank for the formation of a packaging container.

### BRIEF DESCRIPTION OF THE ACCOMPANYING DRAWINGS

The present invention will now be described in greater detail hereinbelow, with the aid of currently preferred embodiments which are shown on the accompanying drawings. In the accompanying drawings:
Fig. 1 schematically illustrates a first embodiment for the stepwise production of a packaging container with low microbiological load, according to the present invention;
Fig. 2 schematically illustrates a second embodiment of a part of the stepwise production in Fig. 1;
Fig. 3 schematically illustrates one example of a packaging container produced according to the present invention;
Fig. 4 schematically illustrates an embodiment of the station B in Fig. 1;
Fig. 5 schematically illustrates an embodiment of a sterilization agent condensation and waste system according to the invention; and
Fig. 6 schematically illustrates a cross section of a condenser used in the system of Fig. 5.

It should be observed that the figures are not according to scale.

### DETAILED DESCRIPTION OF PREFERRED EMBODIMENTS

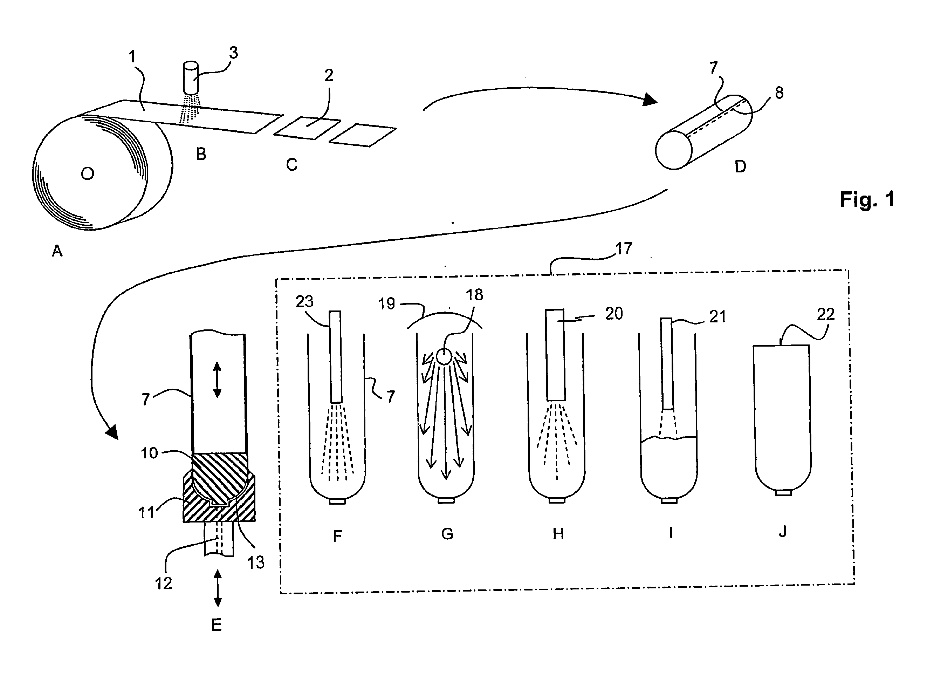

Referring to the drawings, Fig. 1 shows how a web 1 of packaging material is treated and stepwise advanced between different treatment and processing stations in order progressively to be reformed into a packaging container filled with contents and sealed, which may afford a considerably improved shelf-life to the product and which, at least in certain cases, is also suitable for non-refrigerated distribution.

The packaging material is manufactured with a centrally positioned core layer of paper and layers of thin, liquid-tight plastic material, e.g. a thermoplastic such as polyethylene applied on either side thereof. The packaging material may possibly also include additional layers, e.g. layers of gas-tight material such as aluminium foil or some type of barrier plastic, for example a copolymer of ethylene vinyl alcohol (EVOH).

The web-shaped packaging material 1 is initially wound up on a magazine reel which is disposed in the first end of the filling machine, see reference designation A. On production of packaging containers, the material is continuously or intermittently unwound from the reel and cut into sheets 2 of suitable dimensions, see reference designation C. One such sheet is used for the production of one packaging container.

However, before the web 1 is cut into sheets it undergoes a treatment, according to the present invention, with a chemical sterilization agent, see reference designation B. In order for the packaging container, before filling, to have achieved a low microbiological load, the packaging container will undergo in the machine a treatment with a combination of a chemical sterilization agent and UV-light. At B in Fig. 1, the treatment is commenced in that a first partial quantity of the chemical sterilization agent is applied on the packaging material. In such instance, use is preferably made of hydrogen peroxide (H₂O₂) at a concentration of between 0.1 and 5 weight percent, preferably between 0.5 and 3 weight percent. Other conventional sterilization agents may however also be employed.

The hydrogen peroxide which is employed is preferably in gas- or vapour form and is applied on that side of the packaging material which will subsequently form the inside in the future packaging container. The actual application may be put into effect in numerous different ways by means for applying said sterilization agent. In the example described here, the hydrogen peroxide is heated and vaporised to gas phase in a pre-vaporiser in order subsequently to be sprayed out on the web-shaped packaging material using a suitable conventional spraying device 3. The inside of the packaging material will, in such instance, be coated with a thin layer of sterilization agent which first condenses on the surface and is then more or less absorbed into the material.

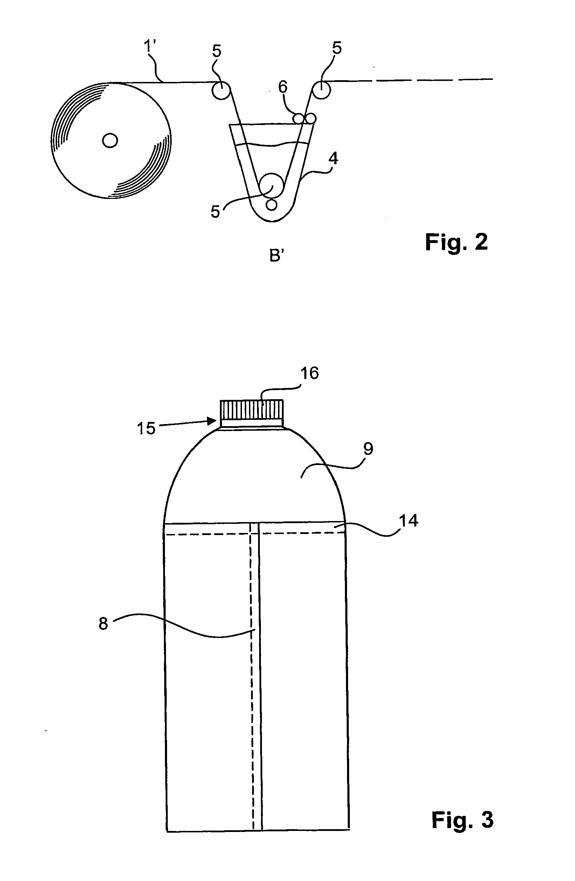

Fig. 2 shows an alternative means for applying, which for simplicity's sake, has been designated B', the first partial quantity of sterilization agent. Here, use is made of a conventional bath 4 which is wholly or partly filled with hydrogen peroxide in liquid form. Preferably, use is also made in this instance of hydrogen peroxide (H₂O₂) at a concentration of between 0.1 and 5 weight percent, preferably between 0.5 and 3 weight percent. The web 1' of packaging material is caused to run through the bath 4 by means of support rollers or cylinders 5 and, on the way up out of the bath 4, surplus hydrogen peroxide is removed so that only a thin layer remains on the surface. This removal may take place using conventional rollers 6 for this purpose.

After pre-treatment using one of the two above described methods and means, the web 1 is cut into sheets 2 as has been described previously.

In means in a station downstream of the cutting means, the sheet 2 is wrapped around a mandrel for forming a tubular packaging container blank 7, see reference designation D. Two longitudinal edges of the sheet are caused to form an overlapped joint 8 which is sealed, see also Fig. 3.

The packaging container blank 7 is then, in one of its open ends, provided with an end wall in the form of a top 9, see Fig. 3, by injection moulding of a thermoplastic material. This takes place by sealing means comprising outer and inner mould halves 10, 11 in a station carrying reference designation E. More precisely, the tubular blank 7 is placed on the inner mould half 10, which is in the form of a mandrel whose end is profiled in a suitable manner in order to form a part of a mould. The blank 7 placed on the inner mould half 10 or the mandrel is thereafter moved, with its one end (subsequently the upper end), into the outer mould half 11 which is preferably of duplex design until its upper edge sealingly closes against the outside of the blank. The mould half 11 has a profiled bottom surface which forms the other half of the mould. Both of the mould halves 10, 11 thus sealingly surround the top end of the tubular packaging container blank 7, and, via a duct or channel 12 in the one mould half 11, a melt of thermoplastic material is thereafter injected. The material fills out the cavity 13 between both of the mould halves 10, 11 and also penetrates out to and encloses the top edges of the tubular packaging container blank, so that a liquid-tight and durable seal 14, see Fig. 3, is formed between the end wall (the top) 9 formed by injection moulding and the packaging container blank 7. In this instance, the profiling of the mould halves may be such that there is created in the end wall a suitable opening device 15. This may, for example, be made in the form of a neck, defining a pouring aperture, the neck being provided with a tear-off membrane. The interface between the membrane and the neck is formed by constrictions in the mould halves 10, 11 which together form a weakening line. In order for the membrane to be able to be removed, it is provided with a pull ring. When the packaging container is sold, the membrane is normally covered by, for instance, a screw cap 16, see Fig. 3. The function of the screw cap 16 is to permit a certain reclosure once the membrane has been torn off. The screw cap 16 has internal threads which cooperate with external threads on the neck, and the packages are normally provided with screw caps when they are finished, i.e. when they are filled and sealed. In one alternative embodiment, the top 9 is injection moulded with a pouring spout or aperture without a membrane, and sealing of the top 9 takes place in that the pouring aperture is provided with a screw cap 16 immediately after the injection moulding operation.

When the tubular packaging container blank 7 has been provided with the end wall 9 by injection moulding, it is moved, by (not shown) conveying means, from the injection moulding station shown at E into a closed space or chamber 17 which surrounds a number of processing or treatment stations for the blank 7 located in sequence after the injection moulding station. The chamber 17, usually entitled a sterile chamber, may in a per se known manner be a completely closed chamber or a chamber provided with controlled outlet, the chamber being continuously supplied with sterile air at excess pressure (possibly heated) in order to ensure that the concentration of bacteria in the chamber is low.

As is apparent from Fig. 1, the chamber 17, in the preferred embodiment of the method according to the invention, comprises five processing or treatment stations.

The first of these is a station F in which the packaging container blank 7 is subjected to a microorganism-reducing treatment in that the second partial quantity of the chemical sterilization agent is supplied to the inside of the packaging container blank. In such instance, use is also preferably made of hydrogen peroxide (H₂O₂) at a concentration of from 0.1 to 5 weight percent, and the practical application preferably takes place, as previously, by partly shown means 23 which is able to vaporise hydrogen peroxide and spray it onto the packaging material. The inside of the packaging container blank will thereby once again be coated with a thin layer of sterilization agent which condenses on the surface. Thus, it will be apparent that the chemical sterilization agent is applied on two occasions, once as early as possible in the filling machine, preferably already when the packaging material is unwound from the magazine reel, and once inside the sterile chamber 17.

After the chemical sterilization agent has been applied in station F, the packaging container blank 7 is displaced to a subsequent station which carries reference designation G. There, the inside of the blank is exposed to irradiation sterilization. The irradiation preferably takes place with UV-light irradiation means in the form of a lamp 18 for UV-light which extends wholly or partly down into the packaging container blank. The lamp 18 may possibly be positioned above the packaging container blank 7 and, in such instance, utilise a reflector 19 in order to ensure that the light beams reach all internal parts of the packaging container blank 7. A conventional UV-lamp can be used and the wavelength range is preferably between 200 and 280 nanometres.

Once the actual microorganism-reducing treatment has been completed, the packaging container blank is displaced to a subsequent station H in which sterile, filtered hot air at a temperature of approx. 200°C is aspirated via an air pipe 20 down into the packaging container blank in order to flush clean the blank from residues of sterilization agent. After a suitable treatment time, the packaging container blank is displaced to a subsequent station, carrying reference designation I, in which filling means comprising a filler pipe 21 supplies the desired contents, e.g. milk, juice or fruit drink, to the packaging container. As soon as the desired volume of contents has been filled into the packaging container, it is displaced a further step to station J, where the packaging container is treated by (not shown) sealing means for sealing, in liquid-tight fashion, its upwardly facing bottom end. This is realised by means of a seal which is put into effect transversely of the longitudinal direction of the tubular blank and which gives rise to the formation of a sealing fin 22. The fin is then folded down and a planar bottom surface is formed by forming and downward folding of flaps. This folding, which is normally designated final folding, however takes place outside the sterile chamber 17 and, in order to facilitate this folding, the packaging material has already initially been provided with crease lines. The crease line pattern and final folding process are naturally selected on the basis of the cross section of the packaging container and will not, in this instance, be described in greater detail.

In practice, the method and device according to the present invention, as described above, has proved to make it possible to produce packaging containers with extended shelf-life with the aid of an extra input of microorganism-reducing type.

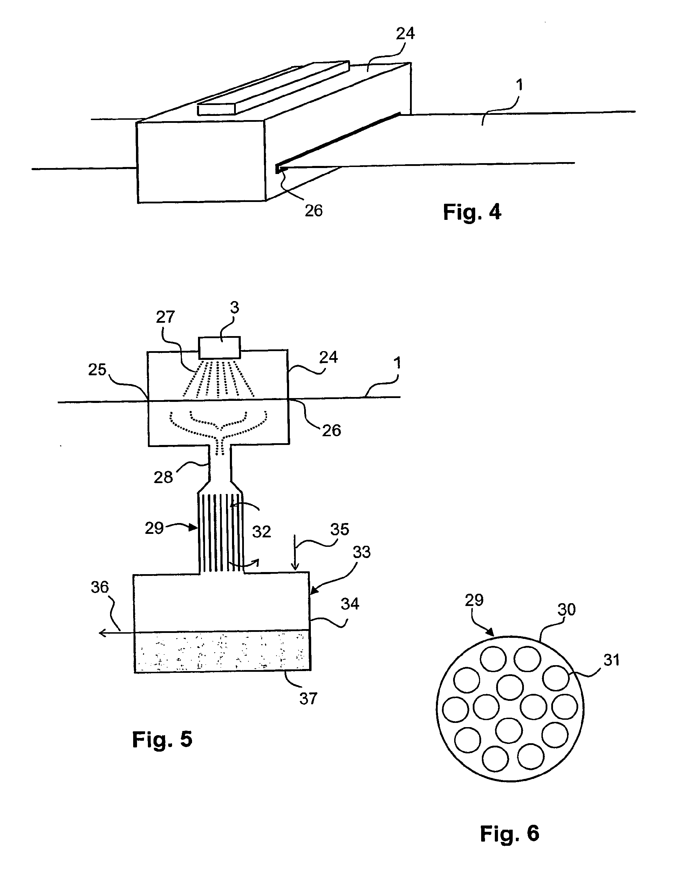

With reference to Fig. 4-6 the station B in Fig. 1 will be further described. In order to minimise the amount of hydrogen peroxide needed and to substantially eliminate spreading of emissions of hydrogen peroxide gas the spraying device 3 in Fig. 1 may preferably be located in a substantially closed chamber. A schematical chamber is shown in Fig. 4 and is denoted 24. Said chamber is provided with an entrance 25 (shown in Fig. 5) and an exit 26 by means of which the web 1 of packaging material is allowed to pass through the chamber 24. In order to minimise the openings in the chamber 24 the entrance and exit are formed as mail slots provided with lip seals (not shown). In the upper wall of the chamber 24 the spraying device 3 is located.

Fig. 5 illustrates a sterilization gent condensation and waste system according to the invention. It is a system for efficiently and easily taking care of the hydrogen peroxide emissions produced when spraying gaseous hydrogen peroxide 27 onto the web 1. As may be understood an amount of the hydrogen peroxide gas that is sprayed will not reach the web 1 and will instead be spread around, but by means of the chamber 24 it will be encapsulated and prevented from spreading to the environment. The chamber is connected to the system, and the system comprises means for condensing the sterilization agent and means for diluting it until a waste product is achieved which may be supplied to the common waste water or sewage system.

The waste hydrogen peroxide is being drained out of the chamber by means of a drainage duct 28. Due to the slight excess pressure being built up in the chamber by the hydrogen peroxide spraying, the hydrogen peroxide waste emissions are automatically forced towards the duct 28. The duct is connected to the condensing means, which is denoted 29 in Figs. 5 and 6. A cross section of said means 29 is shown in Fig. 6 and it can be seen that the means is formed as a pipe 30 comprising several smaller pipes 31. The hydrogen peroxide gas is supplied into the smaller pipes 31. The volume around the smaller pipes, which volume is delimited by the larger pipe 30, is not in fluid connection with the drainage duct 28. However, that volume is connected to a suitable cooling water system for cooling the outer envelope surface of each respective smaller pipe 31. The cooling system is illustrated with the arrows 32 in Fig. 5 and will not be further described.

In the cooled smaller pipes 31 the gaseous hydrogen peroxide is transformed into droplets of liquid, said droplets forming a condensate. The condensate is still at a concentration of between 0.1 and 5 weight percent, depending on the concentration used, and before handling it further it is forwarded to the diluting means. The diluting means is denoted 33. Said means 33 comprises a tank 34 into which the condensate slowly runs from the smaller pipes 31 of the condensing means 29. The tank 34 is provided with a water supply 35 (shown as an arrow) and an overflow valve connected to a waste water pipe, both being illustrated with the arrow denoted 36. Through the supply 35 water, for example city water, is continuously supplied to the condensate in the tank 34 to decrease the concentration of the hydrogen peroxide. In this way the concentration may be lowered to a level permissible for supplying the hydrogen peroxide and water mix 37 to the common waste water or sewage system. The overflow valve 36 regulates the exit of the waste water.

It should be obvious to the skilled person that the present invention is not restricted to the embodiment described in the foregoing, but that a plurality of variations and modifications are conceivable without departing from the scope of the appended claims.

In the described embodiment, the application of the first partial quantity of sterilization agent takes place in a station B after unwinding of the packaging material from the magazine reel. Naturally, it is instead possible to apply the first partial quantity of sterilization agent at a later stage. This depends entirely on the intended effect. Hence, it is possible, for instance, to apply the sterilization agent just before or just after the injection moulding station E, or, for instance, to provide the sterile chamber with two mutually subsequent stations for the application of sterilization agent.

In filling machines which are not supplied by means of a magazine reel of web-shaped packaging material, but which are instead supplied with flat-laid, tubular packaging blanks, the application of the first partial quantity of sterilization agent may suitably take place in a station located at least downstream of a station for raising, i.e. opening up, said initially flat-laid packaging blank. In the case with tubular packaging container blanks, the application of the first partial quantity of the sterilization agent is preferably made by spraying gaseous sterilization agent as has been shown in station B of one of the embodiments.

In the foregoing embodiment example, a packaging container is described as including an injection moulded end wall in the form of a top. It should be understood that the present invention is not restricted to use in connection with this type of packaging container, but that it may of course be employed also in connection with other packaging types. One such packaging type is the type described by way of introduction with a folded and sealed top of the gable top type, which for example is marketed by the applicant under the trademark Tetra Rex®.

Further, in the above described embodiment it is disclosed that the whole of the first partial quantity of sterilization agent is applied in one station. Naturally, it is possible to divide up this quantity among two or more mutually subsequent stations. The same naturally applies to the application of the second partial quantity.

In relation to station B it has been described that the hydrogen peroxide is heated and vaporised to gas phase in a pre-vaporiser and then sprayed out by a suitable conventional spraying device 3. The pre-vaporiser may be of the type in which liquid hydrogen peroxide is sprayed onto a heated surface. Alternatively, an arrangement may be used which comprises a conventional atomizer. Said atomizer sprays a mist of liquid hydrogen peroxide into a sterile air flow being heated above the hydrogen peroxide dew point, thereby vaporising the hydrogen peroxide. Ultrasound nozzles for creating a fine mist of hydrogen peroxide may also be used.

## Claims

1. A method of producing a packaging container with very low microbiological load by treating, in a filling machine, the packaging material with a combination of a chemical sterilization agent and UV-light, **characterised in that**
a first partial quantity of the chemical sterilization agent is applied on the packaging material a sufficiently long time before the packaging material is irradiated with UV-light in order for a predetermined portion of expected microorganisms to have time to absorb a sufficient amount of the chemical sterilization agent so as to be exterminated when irradiated with UV-light; and
that a second partial quantity of the chemical sterilization agent is applied on the packaging material in a sterile chamber (17) before the packaging material is irradiated with UV-light.

2. The method as claimed in claim 1, **characterised in that** the application of said first partial quantity of the chemical sterilization agent takes place outside the sterile chamber (17).

3. The method as claimed in claim 2, **characterised in that** the application of said first partial quantity of the chemical sterilization agent takes place in the very first or one of the first stations in which the packaging material is treated when conveyed through the filling machine.

4. The method as claimed in claim 3, **characterised in that** the filling machine is supplied with at least one magazine reel of web-shaped packaging material (1) and that said first partial quantity of the sterilization agent is applied on the packaging material in the very first or one of the first stations immediately after the unwinding of the packaging material from the magazine reel.

5. The method as claimed in any of the preceding claims 1 to 4, **characterised in that** said first partial quantity of sterilization agent is applied **in that** the packaging material runs through a bath (4) of sterilization agent, and that surplus sterilization agent is removed when the packaging material emerges from said bath (4).

6. The method as claimed in any of the preceding claims 1 to 2, **characterised in that** the filling machine is supplied with flat-laid, tubular packaging blanks and that said first partial quantity of the sterilization agent is applied to the inside of each said packaging blank in a station located at least downstream of a station for raising said packaging blank.

7. The method as claimed in any of the preceding claims 1 to 4 and 6, **characterised in that** said first partial quantity of sterilization agent is applied at least on that side of the packaging material which is to form the inside of the future packaging container, and that said sterilization agent is vaporised before being applied.

8. The method as claimed in any of the preceding claims, **characterised in that** said second partial quantity of sterilization agent is applied on the packaging material in the sterile chamber (17) in a station (F) substantially immediately prior to that station (G) in which the packaging material is irradiated with UV-light.

9. The method as claimed in any of the preceding claims, **characterised in that** the sterilization agent is hydrogen peroxide (H₂O₂) in a concentration in the range of between 0.1 and 5 weight percent.

10. The method as claimed in claim 9, **characterised in that** the hydrogen peroxide is vaporised to gaseous form and applied by spraying, and that the hydrogen peroxide is condensed on the packaging material.

11. The method as claimed in any of the preceding claims, **characterised in that** the UV-light which is employed has a wavelength selected within the range of between 200 and 280 nanometres.

12. The method as claimed in claim 1, **characterised in that**
the filling machine is supplied with at least one magazine reel of web-shaped packaging material (1; 1'),
said first partial quantity of sterilization agent is applied on the inside of the packaging material in a station (B; B') located downstream of a station (A) for unwinding the packaging material (1; 1') from the magazine reel,
the web-shaped packaging material (1; 1') is cut into sheets (2),
each sheet (2) is caused to form a tubular packaging container blank (7),
the one open end of the tubular packaging container blank (7) is closed and sealed **in that** a thin-walled top (9) of thermoplastic is injection moulded on the end,
the packaging container blank (7) is conveyed into the sterile chamber (17),
said second partial quantity of sterilization agent is applied on the inside of the packaging container blank in the sterile chamber (17), and that, in the same chamber, it is ensured that the packaging container blank is irradiated with UV-light, the packaging container blank being filled with the desired contents and the open second end of the packaging container blank is sealed for the formation of a packaging container.

13. Device in a filling machine for producing a packaging container with very low microbiological load, said device being adapted to treat the packaging material with a combination of a chemical sterilization agent and UV-light, **characterised in that** the device comprises
means (3; 4) for applying a first partial quantity of the chemical sterilization agent to the packaging material (1; 1'), and
a sterile chamber (17) comprising means (23) for applying a second partial quantity of the chemical sterilization agent to the packaging material and means (18) for irradiating the packaging material with UV-light, whereby the means (3; 4) for applying the first partial quantity of the chemical sterilization is positioned away from the UV-light irradiating means (18) so that a sufficiently long time will have past before the packaging material is irradiated with UV-light in order for a predetermined portion of expected microorganisms to have absorbed a sufficient amount of the chemical sterilization agent so as to be exterminated when irradiated with the UV-light.

14. The device as claimed in claim 13, **characterised in that** said means (3; 4) for applying the first partial quantity of the chemical sterilization agent is positioned outside the sterile chamber (17).

15. The device as claimed in claim 14, **characterised in that** said means (3; 4) for applying the first partial quantity of the chemical sterilization agent is positioned in the very first or one of the first stations in which the packaging material is treated when conveyed through the filling machine.

16. The device as claimed in claim 15, **characterised in that** the filling machine is supplied with at least one magazine reel of web-shaped packaging material (1) and that said means (3; 4) for applying the first partial quantity of the sterilization agent to the packaging material is positioned in the very first or one of the first stations immediately after the unwinding of the packaging material from the magazine reel.

17. The device as claimed in any of the preceding claims 13 to 16, **characterised in that** said means for applying a first partial quantity of sterilization agent is a bath (4) of sterilization agent through which the packaging material runs, which bath (4) is provided with means (6) adapted to remove surplus sterilization agent when the packaging material emerges from the bath (4).

18. The device as claimed in any of the preceding claims 13 to 14, **characterised in that** the filling machine is supplied with flat-laid, tubular packaging blanks and that said means (3; 4) for applying the first partial quantity of the sterilization agent is positioned in a station located at least downstream of a station for raising said packaging blank, and that said means is adapted to apply said sterilization agent to the inside of each said packaging blank.

19. The device as claimed in any of the preceding claims 13 to 16 and 18, **characterised in that** said means (3) for applying the first partial quantity of sterilization agent comprises means for vaporising the sterilization agent and means for spraying the sterilization agent in gaseous form onto at least that side of the packaging material which is to form the inside of the future packaging container.

20. The device as claimed in any of the preceding claims, **characterised in that** said means (23) for applying the second partial quantity of sterilization agent on the packaging material is positioned in the sterile chamber (17) in a station (F) substantially immediately prior to that station (G) in which the packaging material is irradiated with UV-light.

21. The device as claimed in any of the preceding claims, **characterised in that** the sterilization agent is hydrogen peroxide (H₂O₂) in a concentration in the range of between 0.1 and 5 weight percent.

22. The device as claimed in any of the preceding claims, **characterised in that** the UV-light which is employed has a wavelength selected within the range of between 200 and 280 nanometres.

23. The device as claimed in claim 19, **characterised in that** said means (3) for applying the first partial quantity of sterilization agent is enclosed in a chamber which is connected to a sterilization agent condensation and waste system comprising means for condensing the sterilization agent and means for diluting it.

24. Filling machine comprising a device according to claim 13, **characterised in that**
it is supplied with at least one magazine reel of web-shaped packaging material (1; 1'),
said means (3; 4) for applying the first partial quantity of sterilization agent to the inside of the packaging material is positioned in a station (B) located downstream of a station (A) for unwinding the packaging material from the magazine reel,
means for cutting the web-shaped packaging material (1) into sheets (2),
means for forming each sheet (2) into a tubular packaging container blank (7),
means for closing and sealing the one open end of the tubular packaging container blank (7) by injection moulding a thin-walled top (9) of thermoplastic on said end,
means for conveying the packaging container blank (7) into the sterile chamber (17),
said means (23) for applying the second partial quantity of sterilization agent to the inside of the packaging container blank and the means (18) for irradiating with UV-light are positioned in the sterile chamber (17), and
said sterile chamber (17) being further provided with means (21) for filling the packaging container blank with the desired contents and means for sealing the open second end of the packaging container blank for the formation of a packaging container.

## Patentansprüche

1. Verfahren zum Herstellen eines Verpackungsbehälters mit einer sehr geringen mikrobiologischen Belastung durch Behandeln des Verpackungsmaterials in einer Füllmaschine mit einer Kombination eines chemischen Sterilisierungsmittels mit UV-Licht, **dadurch gekennzeichnet, dass**
eine erste Teilmenge des chemischen Sterilisierungsmittels über eine ausreichend lange Zeit auf das Verpackungsmaterial aufgebracht wird, bevor das Verpackungsmaterial mit UV-Licht bestrahlt wird, um für einen vorbestimmten Anteil von erwarteten Mikroorganismen ausreichend Zeit zu haben, eine ausreichende Menge des chemischen Sterilisierungsmittels zu absorbieren, um bei Bestrahlung mit UV-Licht abgetötet zu werden; und
dass eine zweite Teilmenge des chemischen Sterilisierungsmittels in einer sterilen Kammer (17) auf das Verpackungsmaterial aufgebracht wird, bevor das Verpackungsmaterial mit UV-Licht bestrahlt wird.

2. Verfahren gemäß Anspruch 1, **dadurch gekennzeichnet, dass** das Aufbringen der ersten Teilmenge des chemischen Sterilisierungsmittels außerhalb der sterilen Kammer (17) erfolgt.

3. Verfahren gemäß Anspruch 2, **dadurch gekennzeichnet, dass** das Aufbringen der ersten Teilmenge des chemischen Sterilisierungsmittels in der ersten oder einer der ersten Stationen erfolgt, in denen das Verpackungsmaterial behandelt wird, wenn es durch die Füllmaschine befördert wird.

4. Verfahren gemäß Anspruch 3, **dadurch gekennzeichnet, dass** die Füllmaschine mit wenigstens einer Vorratsrolle von bahnförmigem Verpackungsmaterial (1) versorgt wird und dass die erste Teilmenge des Sterilisierungsmittels in der ersten oder einer der ersten Stationen unmittelbar nach dem Abrollen des Verpackungsmaterials von der Vorratsrolle auf das Verpackungsmaterial aufgebracht wird.

5. Verfahren gemäß einem der vorstehenden Ansprüche 1 bis 4, **dadurch gekennzeichnet, dass** die erste Teilmenge an Sterilisierungsmittel dadurch aufgebracht wird, dass das Verpackungsmaterial durch ein Bad (4) von Sterilisierungsmittel läuft und dass überschüssiges Sterilisierungsmittel entfernt wird, wenn das Verpackungsmaterial aus dem Bad (4) austritt.

6. Verfahren gemäß einem der vorstehenden Ansprüche 1 bis 2, **dadurch gekennzeichnet, dass** die Füllmaschine mit flachgelegten schlauchförmigen Verpackungsrohlingen versorgt wird und dass die erste Teilmenge des Sterilisierungsmittels in einer Station, die einer Station zum Heben des Verpackungsrohlings wenigstens nachgeschaltet angeordnet ist, auf die Innenseite jedes der Verpackungsrohlinge aufgebracht wird.

7. Verfahren gemäß einem der vorstehenden Ansprüche 1 bis 4 und 6, **dadurch gekennzeichnet, dass** die erste Teilmenge von Sterilisierungsmittel auf wenigstens die Seite des Verpackungsmaterials aufgebracht wird, die die Innenseite des zukünftigen Verpackungsbehälters bilden wird, und dass das Sterilisierungsmittel verdampft wird, bevor es aufgebracht wird.

8. Verfahren gemäß einem der vorstehenden Ansprüche, **dadurch gekennzeichnet, dass** die zweite Teilmenge von Sterilisierungsmittel in der sterilen Kammer (17) in einer Station (F) im Wesentlichen unmittelbar vor der Station (G), in der das Verpackungsmaterial mit UV-Licht bestrahlt wird, auf das Verpackungsmaterial aufgebracht wird.

9. Verfahren gemäß einem der vorstehenden Ansprüche, **dadurch gekennzeichnet, dass** das Sterilisierungsmittel Wasserstoffperoxid (H₂O₂) mit einer Konzentration im Bereich zwischen 0,1 und 5 Gewichtprozent ist.

10. Verfahren gemäß Anspruch 9, **dadurch gekennzeichnet, dass** das Wasserstoffperoxid zu gasförmiger Form verdampft und durch Sprühen aufgebracht wird und dass das Wasserstoffperoxid auf dem Verpackungsmaterial kondensiert wird.

11. Verfahren gemäß einem der vorstehenden Ansprüche, **dadurch gekennzeichnet, dass** das UV-Licht, das eingesetzt wird, eine Wellenlänge ausgewählt aus dem Bereich zwischen 200 und 280 Nanometer aufweist.

12. Verfahren gemäß Anspruch 1, **dadurch gekennzeichnet, dass**
die Füllmaschine mit wenigstens einer Vorratsrolle von bahnförmigem Verpackungsmaterial (1; 1') versorgt wird,
die erste Teilmenge von Sterilisierungsmittel in einer Station (B; B'), die einer Station (A) zum Abrollen des Verpackungsmaterials (1; 1') von der Vorratsrolle nachgeschaltet angeordnet ist, auf die Innenseite des Verpackungsmaterials aufgebracht wird,
das bahnförmige Verpackungsmaterial (1; 1') zu Bögen (2) geschnitten wird,
für jeden Bogen (2) bewirkt wird, einen schlauchförmigen Verpackungsbehälterrohling (7) zu bilden,
das eine offene Ende des schlauchförmigen Verpackungsbehälterrohlings (7) verschlossen wird und dadurch versiegelt wird, dass ein dünnwandiges Kopfteil (9) aus Thermoplast auf das Ende gespritzt wird,
der Verpackungsbehälterrohling (7) in die sterile Kammer (17) befördert wird,
in der sterilen Kammer (17) die zweite Teilmenge von Sterilisierungsmittel auf die Innenseite des Verpackungsbehälterrohlings aufgebracht wird und dass in der gleichen Kammer gewährleistet wird, dass der Verpackungsbehälterrohling mit UV-Licht bestrahlt wird, der Verpackungsbehälterrohling mit dem gewünschten Inhalt gefüllt wird und das offene zweite Ende des Verpackungsbehälterrohlings zum Bilden eines Verpackungsbehälters verschlossen wird.

13. Einheit in einer Füllmaschine zum Herstellen eines Verpackungsbehälters mit einer sehr niedrigen mikrobiologischen Belastung, wobei die Einheit dafür gestaltet ist, das Verpackungsmaterial mit einer Kombination eines chemischen Sterilisierungsmittels und UV-Licht zu behandeln, **dadurch gekennzeichnet, dass** die Einheit
Mittel (3; 4) zum Aufbringen einer ersten Teilmenge des chemischen Sterilisierungsmittels auf das Verpackungsmaterial (1; 1') und
eine sterile Kammer (17), umfassend Mittel (23) zum Aufbringen einer zweiten Teilmenge des chemischen Sterilisierungsmittels auf das Verpackungsmaterial und Mittel (18) zum Bestrahlen des Verpackungsmaterials mit UV-Licht, wobei das Mittel (3; 4) zum Aufbringen der ersten Teilmenge der chemischen Sterilisierung von dem Mittel zur Bestrahlung mit UV-Licht (18) entfernt angeordnet ist, so dass eine ausreichend lange Zeit verstrichen sein wird, bevor das Verpackungsmaterial mit UV-Licht bestrahlt wird, damit ein vorbestimmter Anteil von erwarteten Mikroorganismen eine ausreichende Menge des chemischen Sterilisierungsmittels absorbiert hat, um bei Bestrahlung mit dem UV-Licht abgetötet zu werden,
umfasst.

14. Einheit gemäß Anspruch 13, **dadurch gekennzeichnet, dass** das Mittel (3; 4) zum Aufbringen der ersten Teilmenge des chemischen Sterilisierungsmittels außerhalb der sterilen Kammer (17) angeordnet ist.

15. Einheit gemäß Anspruch 14, **dadurch gekennzeichnet, dass** das Mittel (3; 4) zum Aufbringen der ersten Teilmenge des chemischen Sterilisierungsmittels in der ersten oder einer der ersten Stationen angeordnet ist, in denen das Verpackungsmaterial behandelt wird, wenn es durch die Füllmaschine befördert wird.

16. Einheit gemäß Anspruch 15, **dadurch gekennzeichnet, dass** die Füllmaschine mit wenigstens einer Vorratsrolle von bahnförmigem Verpackungsmaterial (1) versorgt ist und dass das Mittel (3; 4) zum Aufbringen der ersten Teilmenge des Sterilisierungsmittels auf das Verpackungsmaterial in der ersten oder einer der ersten Stationen unmittelbar nach dem Abrollen des Verpackungsmaterials von der Vorratsrolle angeordnet ist.

17. Einheit gemäß einem der vorstehenden Ansprüche 13 bis 16, **dadurch gekennzeichnet, dass** das Mittel zum Aufbringen einer ersten Teilmenge von Sterilisierungsmittel ein Bad (4) von Sterilisierungsmittel ist, durch welches das Verpackungsmaterial läuft, welches Bad (4) mit Mitteln (6) versehen ist, die dafür gestaltet sind, überschüssiges Sterilisierungsmittel zu entfernen, wenn das Verpackungsmaterial aus dem Bad (4) austritt.

18. Einheit gemäß einem der vorstehenden Ansprüche 13 bis 14, **dadurch gekennzeichnet, dass** die Füllmaschine mit flachgelegten schlauchförmigen Verpackungsrohlingen versorgt ist und dass das Mittel (3; 4) zum Aufbringen der ersten Teilmenge des Sterilisierungsmittels in einer Station angeordnet ist, die einer Station zum Heben des Verpackungsrohlings wenigstens nachgeschaltet angeordnet ist, und dass das Mittel dafür gestaltet ist, das Sterilisierungsmittel auf die Innenseite jedes der Verpackungsrohlinge aufzubringen.

19. Einheit gemäß einem der vorstehenden Ansprüche 13 bis 16 und 18, **dadurch gekennzeichnet, dass** das Mittel (3) zum Aufbringen der ersten Teilmenge von Sterilisierungsmittel Mittel zum Verdampfen des Sterilisierungsmittels und Mittel zum Sprühen des Sterilisierungsmittels in Gasform auf wenigstens die Seite des Verpackungsmaterials, die die Innenseite des zukünftigen Verpackungsbehälters bilden wird, umfasst.

20. Einheit gemäß einem der vorstehenden Ansprüche, **dadurch gekennzeichnet, dass** das Mittel (23) zum Aufbringen der zweiten Teilmenge von Sterilisierungsmittel auf das Verpackungsmaterial in der sterilen Kammer (17) in einer Station (F) im Wesentlichen unmittelbar vor der Station (G), in der das Verpackungsmaterial mit UV-Licht bestrahlt wird, angeordnet ist.

21. Einheit gemäß einem der vorstehenden Ansprüche, **dadurch gekennzeichnet, dass** das Sterilisierungsmittel Wasserstoffperoxid (H₂O₂) mit einer Konzentration im Bereich zwischen 0,1 und 5 Gewichtprozent ist.

22. Einheit gemäß einem der vorstehenden Ansprüche, **dadurch gekennzeichnet, dass** das UV-Licht, das eingesetzt wird, eine Wellenlänge ausgewählt aus dem Bereich zwischen 200 und 280 Nanometer aufweist.

23. Einheit gemäß Anspruch 19, **dadurch gekennzeichnet, dass** das Mittel (3) zum Aufbringen der ersten Teilmenge von Sterilisierungsmittel in einer Kammer eingeschlossen ist, die mit einem Sterilisierungsmittel-Kondensations-und-Abfallsystem, umfassend Mittel zum Kondensieren des Sterilisierungsmittels und Mittel zum Verdünnen davon, verbunden ist.

24. Füllmaschine, umfassend eine Einheit gemäß Anspruch 13, **dadurch gekennzeichnet, dass**
sie ausgestattet ist mit wenigstens einer Vorratsrolle von bahnförmigem Verpackungsmaterial (1; 1'),
wobei das Mittel (3; 4) zum Aufbringen der ersten Teilmenge von Sterilisierungsmittel auf die Innenseite des Verpackungsmaterials in einer Station (B) angeordnet ist, die einer Station (A) zum Abrollen des Verpackungsmaterials von der Vorratsrolle nachgeschaltet angeordnet ist,
Mittel zum Schneiden des bahnförmigen Verpackungsmaterials (1) zu Bögen (2),
Mittel zum Formen jedes Bogens (2) zu einem schlauchförmigen Verpackungsbehälterrohling (7),
Mittel zum Verschließen und Versiegeln des einen offenen Endes des schlauchförmigen Verpackungsbehälterrohlings (7) durch Spritzgießen eines dünnwandigen Kopfteils (9) aus Thermoplast auf das Ende,
Mittel zum Befördern des Verpackungsbehälterrohlings (7) in die sterile Kammer (17),
wobei das Mittel (23) zum Aufbringen der zweiten Teilmenge von Sterilisierungsmittel auf die Innenseite des Verpackungsbehälterrohlings und das Mittel (18) zum Bestrahlen mit UV-Licht in der sterilen Kammer (17) angeordnet sind, und
die sterile Kammer (17) ferner mit Mitteln (21) zum Füllen des Verpackungsbehälterrohlings mit dem gewünschten Inhalt und Mitteln zum Versiegeln des offenen zweiten Endes des Verpackungsbehälterrohlings zum Bilden eines Verpackungsbehälters ausgestattet ist.

## Revendications

1. Procédé destiné à produire un récipient de conditionnement à très faible charge microbiologique en traitant, dans une machine de remplissage, le matériau de conditionnement à l'aide d'une combinaison d'un agent de stérilisation chimique et d'une lumière UV, **caractérisé en ce que**
une première quantité partielle de l'agent de stérilisation chimique est appliquée sur le matériau de conditionnement suffisamment longtemps avant que le matériau de conditionnement soit irradié par la lumière UV pour qu'une partie prédéterminée de micro-organismes prévus ait le temps d'absorber une quantité suffisante de l'agent de stérilisation chimique de façon à être exterminés lorsqu'ils sont irradiés par la lumière UV ; et
**en ce qu'**une deuxième quantité partielle de l'agent de stérilisation chimique est appliquée sur le matériau de conditionnement dans une chambre stérile (17) avant que le matériau de conditionnement soit irradié par la lumière UV.

2. Procédé selon la revendication 1, **caractérisé en ce que** l'application de ladite première quantité partielle de l'agent de stérilisation chimique a lieu à l'extérieur de la chambre stérile (17).

3. Procédé selon la revendication 2, **caractérisé en ce que** l'application de ladite première quantité partielle de l'agent de stérilisation chimique a lieu sur le tout premier ou l'un des premiers postes sur lesquels le matériau de conditionnement est traité lorsqu'il est acheminé à travers la machine de remplissage.

4. Procédé selon la revendication 3, **caractérisé en ce que** la machine de remplissage est alimentée par au moins un rouleau chargeur de matériau (1) de conditionnement en forme de bande et **en ce que** ladite première quantité partielle de l'agent de stérilisation est appliquée sur le matériau de conditionnement sur le tout premier ou l'un des premiers postes immédiatement après le déroulage du matériau de conditionnement à partir du rouleau chargeur.

5. Procédé selon l'une quelconque des revendications précédentes 1 à 4, **caractérisé en ce que** ladite première quantité partielle d'agent de stérilisation est appliquée au sens où le matériau de conditionnement passe à travers un bain (4) d'agent de stérilisation, et **en ce que** l'agent de stérilisation excédentaire est éliminé lorsque le matériau de conditionnement émerge dudit bain (4).

6. Procédé selon l'une quelconque des revendications précédentes 1 à 2, **caractérisé en ce que** la machine de remplissage est alimentée en flans tubulaires de conditionnement posés à plat et **en ce que** ladite première quantité partielle de l'agent de stérilisation est appliquée à l'intérieur de chacun desdits flans de conditionnement sur un poste situé au moins en aval d'un poste servant à élever ledit flan de conditionnement.

7. Procédé selon l'une quelconque des revendications précédentes 1 à 4 et 6, **caractérisé en ce que** ladite première quantité partielle d'agent de stérilisation est appliquée au moins sur le côté du matériau de conditionnement qui est appelé à former l'intérieur du futur récipient de conditionnement, et **en ce que** ledit agent de stérilisation est vaporisé avant d'être appliqué.

8. Procédé selon l'une quelconque des revendications précédentes, **caractérisé en ce que** ladite deuxième quantité partielle d'agent de stérilisation est appliquée sur le matériau de conditionnement dans la chambre stérile (17) sur un poste (F) précédant sensiblement immédiatement le poste (G) sur lequel le matériau de conditionnement est irradié par la lumière UV.

9. Procédé selon l'une quelconque des revendications précédentes, **caractérisé en ce que** l'agent de stérilisation est du peroxyde d'hydrogène (H₂O₂) à une concentration située dans la plage comprise entre 0,1 et 5 pour cent en poids.

10. Procédé selon la revendication 9, **caractérisé en ce que** le peroxyde d'hydrogène est vaporisé pour passer sous forme gazeuse et appliqué par pulvérisation, et **en ce que** le peroxyde d'hydrogène est condensé sur le matériau de conditionnement.

11. Procédé selon l'une quelconque des revendications précédentes, **caractérisé en ce que** la lumière UV qui est employée présente une longueur d'onde choisie dans la plage comprise entre 200 et 280 nanomètres.

12. Procédé selon la revendication 1, **caractérisé en ce que**
la machine de remplissage est alimentée par au moins un rouleau chargeur de matériau de conditionnement (1 ; 1') en forme de bande,
ladite première quantité partielle d'agent de stérilisation est appliquée sur l'intérieur du matériau de conditionnement sur un poste (B ; B') situé en aval d'un poste (A) servant à dérouler le matériau de conditionnement (1 ; 1') à partir du rouleau chargeur,
le matériau (1 ; 1') de conditionnement en forme de bande est découpé en feuilles (2),
il est fait en sorte que chaque feuille (2) forme un flan tubulaire (7) pour récipient de conditionnement,
l'extrémité ouverte du flan tubulaire (7) pour récipient de conditionnement est fermée et scellée au sens où un dessus (9) à paroi mince en thermoplastique est moulé par injection sur l'extrémité,
le flan (7) pour récipient de conditionnement est acheminé jusque dans la chambre stérile (17),
ladite deuxième quantité partielle d'agent de stérilisation est appliquée sur l'intérieur du flan pour récipient de conditionnement dans la chambre stérile (17), et
**en ce que**, dans la même chambre, on s'assure que le flan pour récipient de conditionnement est irradié par la lumière UV, le flan pour récipient de conditionnement étant rempli du contenu souhaité et la deuxième extrémité ouverte du flan pour récipient de conditionnement étant scellée pour la formation d'un récipient de conditionnement.

13. Dispositif dans un machine de remplissage destinée à produire un récipient de conditionnement à très faible charge microbiologique, ledit dispositif étant prévu pour traiter le matériau de conditionnement à l'aide d'une combinaison d'un agent de stérilisation chimique et d'une lumière UV, **caractérisé en ce que** le dispositif comporte
des moyens (3 ; 4) servant à appliquer une première quantité partielle de l'agent de stérilisation chimique au matériau (1 ; 1') de conditionnement, et
une chambre stérile (17) comportant un moyen (23) servant à appliquer une deuxième quantité partielle de l'agent de stérilisation chimique au matériau de conditionnement et un moyen (18) servant à irradier le matériau de conditionnement par une lumière UV, les moyens (3 ; 4) servant à appliquer la première quantité partielle de l'agent de stérilisation chimique étant ainsi positionnés à l'écart du moyen (18) d'irradiation par lumière UV de telle façon qu'un temps suffisamment long se soit écoulé avant que le matériau de conditionnement soit irradié par la lumière UV pour qu'une partie prédéterminée de micro-organismes prévus air absorbé une quantité suffisante de l'agent de stérilisation chimique de façon à être exterminés lorsqu'ils sont irradiés par la lumière UV.

14. Dispositif selon la revendication 13, **caractérisé en ce que** lesdits moyens (3 ; 4) servant à appliquer la première quantité partielle de l'agent de stérilisation chimique sont positionnés à l'extérieur de la chambre stérile (17).

15. Dispositif selon la revendication 14, **caractérisé en ce que** lesdits moyens (3 ; 4) servant à appliquer la première quantité partielle de l'agent de stérilisation chimique sont positionnés sur le tout premier ou l'un des premiers postes sur lesquels le matériau de conditionnement est traité lorsqu'il est acheminé à travers la machine de remplissage.

16. Dispositif selon la revendication 15, **caractérisé en ce que** la machine de remplissage est alimentée par au moins un rouleau chargeur de matériau (1) de conditionnement en forme de bande et **en ce que** lesdits moyens (3 ; 4) servant à appliquer la première quantité partielle de l'agent de stérilisation au matériau de conditionnement sont positionnés sur le tout premier ou l'un des premiers postes immédiatement après le déroulage du matériau de conditionnement à partir du rouleau chargeur.

17. Dispositif selon l'une quelconque des revendications précédentes 13 à 16, **caractérisé en ce que** lesdits moyens servant à appliquer une première quantité partielle d'agent de stérilisation sont un bain (4) d'agent de stérilisation à travers lequel passe le matériau de conditionnement, ledit bain (4) étant muni d'un moyen (6) prévu pour éliminer l'agent de stérilisation excédentaire lorsque le matériau de conditionnement émerge du bain (4).

18. Dispositif selon l'une quelconque des revendications précédentes 13 à 14, **caractérisé en ce que** la machine de remplissage est alimentée en flans tubulaires de conditionnement posés à plat et **en ce que** lesdits moyens (3 ; 4) servant à appliquer la première quantité partielle de l'agent de stérilisation sont positionnés sur un poste situé au moins en aval d'un poste servant à élever ledit flan de conditionnement, et **en ce que** lesdits moyens sont prévus pour appliquer ledit agent de stérilisation à l'intérieur de chacun desdits flans de conditionnement.

19. Dispositif selon l'une quelconque des revendications précédentes 13 à 16 et 18, **caractérisé en ce que** ledit moyen (3) servant à appliquer la première quantité partielle d'agent de stérilisation comporte un moyen servant à vaporiser l'agent de stérilisation et un moyen servant à pulvériser l'agent de stérilisation sous forme gazeuse sur au moins le côté du matériau de conditionnement qui est appelé à former l'intérieur du futur récipient de conditionnement.

20. Dispositif selon l'une quelconque des revendications précédentes, **caractérisé en ce que** ledit moyen (23) servant à appliquer la deuxième quantité partielle d'agent de stérilisation sur le matériau de conditionnement est positionné dans la chambre stérile (17) sur un poste (F) précédant sensiblement immédiatement le poste (G) sur lequel le matériau de conditionnement est irradié par la lumière UV.

21. Dispositif selon l'une quelconque des revendications précédentes, **caractérisé en ce que** l'agent de stérilisation est du peroxyde d'hydrogène (H₂O₂) à une concentration située dans la plage comprise entre 0,1 et 5 pour cent en poids.

22. Dispositif selon l'une quelconque des revendications précédentes, **caractérisé en ce que** la lumière UV qui est employée présente une longueur d'onde choisie dans la plage comprise entre 200 et 280 nanomètres.

23. Dispositif selon la revendication 19, **caractérisé en ce que** ledit moyen (3) servant à appliquer la première quantité partielle d'agent de stérilisation est enfermé dans une chambre qui est reliée à un système de condensation et de rejet d'agent de stérilisation comportant un moyen servant à condenser l'agent de stérilisation et un moyen servant à le diluer.

24. Machine de remplissage comportant un dispositif selon la revendication 13, **caractérisée**
**en ce qu'**elle est dotée d'au moins un rouleau chargeur de matériau (1 ; 1') de conditionnement en forme de bande,
**en ce que** lesdits moyens (3 ; 4) servant à appliquer la première quantité partielle d'agent de stérilisation à l'intérieur du matériau de conditionnement sont positionnés sur un poste (B) situé en aval d'un poste (A) servant à dérouler le matériau de conditionnement à partir du rouleau chargeur,
un moyen servant à découper en feuilles (2) le matériau (1) de conditionnement en forme de bande,
un moyen servant à mettre chaque feuille (2) sous la forme d'un flan tubulaire (7) pour récipient de conditionnement,
un moyen servant à fermer et à sceller l'extrémité ouverte du flan tubulaire (7) pour récipient de conditionnement en moulant par injection un dessus (9) à paroi mince en thermoplastique sur ladite extrémité,
un moyen servant à acheminer le flan (7) pour récipient de conditionnement jusque dans la chambre stérile (17), ledit moyen (23) servant à appliquer la deuxième quantité partielle d'agent de stérilisation à l'intérieur du flan pour récipient de conditionnement et le moyen (18) servant à irradier par une lumière UV étant positionnés dans la chambre stérile (17), et
ladite chambre stérile (17) étant en outre munie d'un moyen (21) servant à remplir le flan pour récipient de conditionnement du contenu souhaité et d'un moyen servant à sceller la deuxième extrémité ouverte du flan pour récipient de conditionnement en vue de la formation d'un récipient de conditionnement.
